Europäisches Patentamt

European Patent Office

Office européen des brevets

(19)

(11) Numéro de publication: **0 298 820**
**A1**

# DEMANDE DE BREVET EUROPEEN

(12)

(21) Numéro de dépôt: **88401592.6**

(22) Date de dépôt: **23.06.88**

(51) Int. Cl.4: **C 07 K 7/06**
A 61 K 37/02, C 12 Q 1/56,
G 01 N 33/58

(30) Priorité: **25.06.87 FR 8708983**

(43) Date de publication de la demande:
**11.01.89 Bulletin 89/02**

(84) Etats contractants désignés:
**AT BE CH DE ES FR GB GR IT LI LU NL SE**

(71) Demandeur: **INSTITUT NATIONAL DE LA SANTE ET DE LA RECHERCHE MEDICALE (INSERM)**
**101, rue de Tolbiac**
**F-75654 Paris Cédex 13 (FR)**

**LABORATOIRE L. LAFON**
**19 Avenue du Professeur Cadiot**
**F-94701 Maisons Alfort (FR)**

(72) Inventeur: **Marguerie de Rotrou, Gérard Armand**
**5 Avenue Albert 1er de Belgique**
**F-38000 Grenoble (FR)**

(74) Mandataire: **Le Guen, Gérard et al**
**CABINET LAVOIX 2, place d'Estienne d'Orves**
**F-75441 Paris Cédex 09 (FR)**

Une requête en rectification du tableau 2 ("Gla" doit être "Gln" dans séquence du premier peptide) a été présentée conformément à la règle 88 CBE. Il est statué sur cette requête au cours de la procedure engagée devant la division d'examen (Directives relatives à l'examen pratiqué à l'OEB, A-V, 2.2).

(54) **Nouveaux dérivés peptidiques et leur application notamment en thérapeutique.**

(57) L'invention a pour objet des dérivés peptidiques de formule

X- Lys - Gln - Y - Gly - Asp - Z - W     (I)

dans laquelle X représente l'hydrogène, un résidu d'acide aminé, un groupement de formule DE-où D représente un groupe N-protecteur physiologiquement acceptable, ou l'hydrogène, et où E représente une simple liaison ou un résidu d'acide aminé.

Y représente un résidu L-arginye, ou D-arginyle;

Z représente un résidu L-valyle, D-valyle, L-phénylalanyle, D-phénylalanyle, L-séryle, ou D-séryle,

W représente un groupe OH, $NH_2$, $O-R^1$ avec $R^1$ représentant un radical alkyle en $C_1$ à $C_4$ $NHR^2$ avec $R^2$ représentant un radical alkyle en $C_1$ à $C_4$, ou un résidu d'acide aminé.

EP 0 298 820 A1

**Description**

## Nouveaux dérivés peptidiques et leur application notamment en thérapeutique

La présente invention concerne de nouveaux dérivés peptidiques ayant une activité antiagrégante, leur procédé de préparation et leurs applications en thérapeutique et comme agents de diagnostic.

De façon plus précise, elle concerne des analogues peptidiques d'une séquence du fibrinogène utilisables notamment comme antagonistes du fibrinogène vis à vis des plaquettes sanguines.

On sait qu'au cours de l'hémostase les plaquettes sanguines adhèrent au sous-endothélium du vaisseau lésé, secrètent leur contenu granulaire après stimulation et agrègent les unes aux autres pour former un thrombus plaquettaire. L'agrégation dépend des contacts qui s'établissent entre membranes de plaquettes adjacentes. Cette réaction est nécessaire pour l'arrêt d'un saignement. Elle a cependant de nombreuses déviations pathologiques, notamment dans les cas de thromboses veineuses ou artérielles, au cours du développement d'une plaque d'athérome, ou de la formation de microthrombi qui peuvent obstruer la micro-circulation périphérique ou cérébrale. Le contrôle et la régulation de l'agrégation plaquettaire sont donc un objectif majeur dans la prévention de la thrombose et de l'athérosclérose et de nombreuses études sont consacrées à la recherche et au développement de molécules aux propriétés antiagrégantes.

Dans ce phénomène, le fibrinogène a un rôle important. Ainsi dans le plasma de malades atteints d'afibrinogènémie congénitale, l'agrégation plaquettaire est fortement diminuée ou absente et ce défaut est corrigé par l'injection de fibrinogène. De même en l'absence de fibrinogène, des plaquettes lavées n'agrègent pas à l'ADP ou à l'épinéphrine. La présence de fibrinogène est donc une nécessité pour le dévelop pement normal d'un thrombus plaquettaire. La participation du fibrinogène à l'agrégation est due à l'induction d'un récepteur spécifique pour cette protéine sur la membrane de la plaquette activée. Tous les stimuli physiologiques de la plaquette induisent une classe unique de récepteur et l'interaction du fibrinogène avec ce récepteur régule l'agrégation plaquettaire. Il existe donc un mécanisme de l'agrégation plaquettaire, commun à tous les inducteurs, qui dépend de l'interaction entre le fibrogène et son récepteur. L'importance physiologique de cette voie de l'agrégation plaquettaire dépendante du fibrinogène est attestée par l'étude des thrombasthénies de Glanzmann dont les plaquettes ne fixent pas le fibrinogène et n'agrègent pas en réponse à tous les stimuli physiologiques de la cellule.

En résumé, le récepteur du fibrinogène n'est pas exprimé sur la plaxquette circulante, il est induit dès que la cellule est stimulée. Cette induction peut être dépendante ou indépendante de la réaction de sécrétion. Tous les stimuli expriment le même récepteur et l'interaction entre le fibrinogène et le récepteur conduit directement à l'agrégation. La dissociation du fibrinogène lié à la plaquette a pour conséquence la désagrégation des plaquettes.

Dans ce contexte il est clair que si l'interaction entre le fibrinogène et son récepteur plaquettaire pouvait être régulée, ceci constituerait un moyen pour contrôler l'agrégation in vitro et in vivo.

La présente invention vise précisément à fournir de nouveaux agents qui permettent d'inhiber, de réguler ou de mesurer sélectivement la voie de l'agrégation dépendante du fibrinogène.

Deux séquences peptidiques issues de la molécule de fibrinogène ont déjà été indentifiées comme inhibant la fixation de cette protéine sur les plaquettes et bloquant ainsi leur agrégation :
- une séquence Arg-Gly-Asp-Ser (RGDS) [1]présente dans la chaîne du fibrinogène (E. Plow et coll. Proc. Natl. Acad. Sci. USAC 82 8057, 1985)
- un dodécapeptide $H_{12}$ (H-H-L-G-G-A-K-Q-A-G-D-V) représentant l'extrémité C-terminale de la chaîne du fibrinogène (E. Plow et coll. J. Biol. Chem., 259-, 5388,1984).

La présente invention a pour objet des analogues peptidiques de l'extrémité C-terminale de la chaîne du fibrinogène qui présentent un effet inhibiteur de la liaison fibrinogène-plaquette et de l'agrégation plaquettaire.

Ces dérivés peptidiques répondent à la formule générale suivante :

X- Lys - Gln - Y - Gly - Asp - Z - W      (I)

dans laquelle X représente l'hydrogène, un résidu d'acide aminé, un groupement de formule DE-où D représente un groupe N-protecteur physiologiquement acceptable, ou l'hydrogène, et où E représente une simple liaison ou un résidu d'acide aminé,

Y représente un résidu L-arginyle, ou D-arginyle,

Z représente un résidu L-valyle, D-valyle, L-phénylalanyle, D-phénylalanyle, L-séryle, ou D-séryle,

W représente un groupe OH, $NH_2$, O-$R^1$ avec $R^1$ représentant un radical alkyle en $C_1$ à $C_4$ NH$R^2$ avec $R^2$ représentant un radical alkyle en $C_1$ à $C_4$, ou un résidu d'acide aminé.

Dans les groupes X et W les résidus d'acides aminés susceptibles d'être utilisés sont notamment les radicaux D ou L-pyroglutamyle, L ou D-alanyle, **glycycle,**L ou D-prolyle, L ou D-valyle, L ou D-phénylalanyle, L ou D-homocystéyle, L ou D-aspartyle, L ou D-glutamyle, L ou D-hystidyle, L ou D-méthionyle, L ou D-thréonyle, L ou D-séryle, L ou D-cystéyle, L ou D-leucyle, L ou D-arginyle, L ou D-tryptophanyle, L ou D-tyrosyle, L ou D-lysyle et L ou D-ornithyle.

Les groupes N-protecteurs physiologiquement acceptables sont notamment les groupes protecteurs de l'attaque en N-terminal des enzymes exopeptidases. Comme exemples de tels groupes on peut citer les groupes acyles tels que les groupes t-butyloxycarbonyle (Boc), tert-amyl-oxycarbonyle ($^+$Aoc), benzyloxycarbonyle, benzoyle, acétyle, formyle, propanoyle, butanoyle, phénylacétyle, phenylpropanoyle, cyclo pentylcar-

[1] La signification des symboles et abréviations utilsees est donnée en annexe.

bonyle.

La présente invention englobe également les équivalents des dérivés peptidiques de formule I dans lesquels chaque liaison peptidique (-CO-NH-) entre deux résidus d'acide aminé de la formule générale I est remplacée par les structures suivantes :

- CO-N(CH$_3$)- ; -CO-O- ; -CH$_2$-NH- ; -CS-NH- ;
- CO-CH$_2$ ; CH$_2$-S- ; -CHOH-CH$_2$- ; -HN-CO- ;
- CH=CH- ; - CH$_2$-CH$_2$-.

ou dans lesquels le squelette peptidique présente un ou plusieurs groupes intercalés tels que des groupes -CH$_2$-, -NH-, -O-.

Un dérivé peptidique préféré est un dérivé de formule

H -$_L$Lys-$_L$Gln-$_L$Arg-Gly-$_L$Asp-$_L$Phe-OH

La présente invention a également pour objet
- une composition pharmaceutique comprenant à titre de principe actif un dérivé peptidique de formule I
-un agent de diagnostic comprenant un dérivé de peptidique de formule I.

Les dérivés peptidiques de formule I peuvent être préparés de manière classique par synthèse peptidique en phase liquide ou solide par couplages successifs des différents résidus d'acides aminés à incorporer (de l'extrémité N-terminale vers l'extrémité C-terminale en phase liquide, ou de l'extrémité C-terminale vers l'extrémité N-terminales en phase solide) et dont les extrémités N-terminales et les chaînes latérales réactives sont préalablement bloquées par des groupements tels que ceux mentionnés ci-dessous :

1) Extrémité N-terminale protégée par :

Boc
Bpoc
Fmoc

2)

| Résidu | Groupement bloquant la chaîne latérale |
|--------|----------------------------------------|
| Alanyle | H |
| Arginyle | tosyle |
| Asparagyle | H,xanthyle |
| Aspartyle | O-benzyle |
| Cystéyle | acétamidométhyle(Acm), 4-méthylbenzyle (Meb), 4-méthoxybenzyle(Mob), S-ben- zyle |
| Glutamyle | O-benzyle |
| Glutaminyle | xanthyle |
| Glycyle | H |
| Histydyle | tosyle, 2-4-dinitrophényle(Dnp) |
| Isoleucyle | H |
| Leucyle | H |
| Lysyle | 2-chlorobenzyloxycarbonyle(Clz), tri- fluoroacétyle(TFA), formyle(For), ben- zyloxycarbonyle(Z) |
| Méthionyle | H |
| Norleucyle | H |
| Ornithyle | benzyloxycarbonyle |
| Phénylalanyle | H |
| Propyle | H |
| Pyroglutamyle | H |
| Sarcosyle | H |
| Séryle | O-benzyle |
| Thréonyle | H, O-benzyle |
| Tryptophanyle | H, formyle |
| Tyrosyle | H, 2-6-dichlorobenzyle(dcb), 2-bromobenzyloxycarbonyle |
| Valyle | H |

On peut utiliser différentes méthodes de couplage :

1. Couplage des résidus par un carbodiimide (ex : DCC, EDC) avec ou sans catalyse (ex : HOBT) ou autre agent couplant (ex : EEDQ)

2. Utilisation des acides aminés sous forme d'anhydrides symétriques préformés

3. Utilisation des acides aminés sous forme d'esters activés (ex : p-nitrophénylester, HOBT ester) et couplage par l'intermédiare de DCC.

En synthèse en phase solide (SPPS) le tableau I ci-dessous mentionne les différents types de résine utilisable ainsi que les protections et méthodes adéquates pour les différentes étapes.

3

## Tableau I

| Système SPPS utilisé | Liaison peptide-résine | Protection en alpha | réactif de déprotection | protection chaine latérale | réactif de clivage |
|---|---|---|---|---|---|
| Classique | Benzyl ester | Boc | TFA, HCl | Benzyl | HF, HBr |
| Stable (longue chaine) | Pam | Boc | TFA, HCl | Benzyl | HF, HBr |
| | Benzyl ester | Bpoc | TFA dilué | Benzyl | HF |
| | Benzyl ester | Bpoc | TFA dilué | t-Butyl | HF |
| Labile | Ether résine | Bpoc | TFA dilué | t-Butyl | TFA |
| Orthogonal | Ether résine | Fmoc | Piperidine | t-Butyl | TFA |
| Synthèse de segment | Ether résine | Fmoc | Piperidine | Benzyl | TFA |
| | t-Butyl résine | Fmoc | Piperidine | Benzyl | TFA |
| | Hydrazide résine | Fmoc | Piperidine | Benzyl | TFA |
| Assemblement de segments | Benzyl ester | Fmoc | Piperidine | Benzyl | HF |
| Peptides amides | MBHA, BHA | Boc | TFA, HCl | Benzyl | HF |
| Peptides alcools | Benzyl ester | Boc | TFA, HCl | Benzyl | LiBH$_4$ |

En synthèse en phase solide le 1er acide aminé (extrémité C-terminale) à fixer sur la résine peut être soit acquis commercialement déjà attaché au support soit fixé par l'intermédiaire de sel de césium (méthode de Gisin), d'un sel de tétraméthylammonium (méthode de Loffet) ou d'un carbodiimide.

Les exemples suivants illustrent la préparation des dérivés peptidiques de formule I

Exemple 1 :

Synthèse du peptide
H-Ala-Lys-Gln-Arg-Gly-Asp-Val-OH (ou A K Q R G D V, également dénommé A$_7$ (R$_4$)

Ce peptide a été synthétisé en phase solide en utilisant les méthodes suivantes :
- support : résine Merrifield chlorométhylée (0.7 mmol Cl/g) à 2% de divinylbenzène.

Préparation de Boc-Phe-résine :

10 mmoles (2g) de Boc-Valine solubilisées dans 10 ml d'éthanol ont été additionnées de 5 mmoles (1,63g) de Cs$_2$CO$_3$ dans 2 ml d'eau. L'ester de césium formé (Boc-Val-OCs, 10 mmol) après quelques minutes d'agitation a été évaporé à sec et mis au dessicateur sous vide pendant 48 h. Il a été ensuite resolubilisé par 40 ml de DMF et mélangé à 15g de résine (10,5 mmol Cl). La réaction a été réalisée en 24 h. sous agitation dans un bain chauffant à 50°C. La résine dérivatisée (Boc-Val-résine) a été ensuite filtrée et lavée par DMF, DMF/H$_2$O, DMF, EtOH, puis séché sous vide pendant 3 h. Le taux de substitution de la résine a été calculé par analyse d'acide aminé après hydrolyse à 150°C, pendant 3 h., par le mélange HCl/acide propionique (50/50). La substitution obtenue a été de 0,34 mmol Val/g résine.
- couplage :
Tous les dérivés aminés incorporés ont été couplés par la méthode du carbodiimide DCC en présence de catalyseur HOBT (2 équivalents Val en acide aminé, en DCC et en HOBT).
Les dérivés protégés utilisés et leur milieu de solubilisation ont été les suivants :

4

| Dérivés | Solvants |
|---|---|
| Boc-Asp-O-Bzl | DCM |
| Boc-Gly | DCM |
| Boc-Arg(Tos) | DMF |
| Boc-Gln(Xab) | DMF |
| Boc-Lys(Clz) | DCM |
| Boc-Ala | DMC |

Les couplages ont été réalisés en 2 h. dans du DCM.

Les déprotections par TFA et les couplages ont tous été contrôlés par un test de Kaiser

- clivage :

Le clivage du peptide synthétisé a été effectué par HF (10 ml/g résine) en présence d'anisol (1 ml/g rés.) pendant 1 h. à 0°C. Après lavage à l'éther, le peptide a été ensuite extrait par de l'acide acétique à 15% et lyophilisé.

- purification : sur colonne de Sephadex G10 éluée par de l'acide acétique à 10%
- contrôle :

. analyse d'acide aminé : après hydrolyse pendant 30 mn à 150°C par HCl/acide propionique 50/50

. chromatographie sur couche mince de silice :

éluant : CH₃OH/CHCl₃/NH₄OH 25% (60/40/20)

détection :

phénantrène quinone (Arg)

ninhydrine (NH₂)

TDM (NH)

$R_f = 0,22$

-HPLC : sur colonne analytique de phase inverse C18

éluant : gradient H₂O/acétonitrile (100/0 à (20/80) en 25 nm à ml/nm

détection : absorption optique à 210 nm.

$T_r = 0,6$ nm.

Exemple 2 :

Synthèse du peptide :

H-Lys-Gln-Arg-Gly-Asp-Val-OH (ou K Q R G D V, également dénommé K₆ (R₄).

Ce peptide a été synthétisé, purifié et analysé selon les mêmes méthodes que le peptide de l'exemple 1.

Ses caractéristiques analytiques sont les suivantes :

. chromatographie sur couche mince de silice :

éluant 1 :CH₃OH/CHCl₃/NH₄OH 25% (60/40/20)

$R_f = 0,12$

éluant 2 : 2 - butanone/CH₃COOH/H₂O (10/30/25)

$R_f - 0,69$

-HPLC :

$T_r = 1,2$ mn.

Exemple 3 :

Synthèse du peptide :

H-Lys-Gln-Arg-Gly-Asp-Phe-OH (ou K Q R G D F, également dénommé K₆ (R₄F₁)).

Ce peptide a été synthétisé, purifié et analysé selon les mêmes méthodes que le peptide de l'exemple 1.

Ces caractéristiques en chromatographie en couche mince sont les suivantes :

éluant 1 :CH₃OH/CHCl₃/NH₄OH 25% (60/40/20)

$R_f = 0,51$

éluant 2 = 2 - butanone/CH₃COOH/H₂O (10/30/25)

$R_f - 0,75$

On donnera ci-après des résultats des études pharmacologiques mettant en évidence les propriétés des dérivés peptidiques de formule I

## 1. Inhibition de la liaison plaquette-fibrinogène

### Préparation des plaquettes

Les plaquettes sont isolées à partir de 60 ml de sang humain prélevé sur un tampon anticoagulant, l'ACD, à raison d'1 volume d'ACD pour 6 volumes de sang.

L'ACD a la composition suivante :

Citrate trisodique 5 $H_2O$     5,95 g

Acide citrique     3,41 g

Dextrose     5 g

$H_2O$     qsp 250 ml

Le sang est ensuite centrifugé 20 mn à 1000 t/mn (centrifugeuse JOUAN E 96) à température ambiante.

Le PRP (plasma riche en plaquettes) est décanté et additionné de $PGE_1$ 0,1 uM puis centrifugé 15 mn à 2000 t/mn.

Les plaquettes obtenues dans le culot sont alors reprises par 1 ml de tampon Tyrode-albumine pH 7,2 préparé selon la composition suivante :

Tampon Tyrode (solution mère) :

NaCl     1,3 M

KCl     0,026 M

$NaHCO_3$     0,12 M

Tampon Tyrode-albumine :

solution mère     1/10 M

D-glucose     0,0055 M

albumine     2 %

HCl 1M     qsp pH 7,2

Les plaquettes sont lavées sur une colonne de Sépharose CL 2B par du tampon tyrode-albumine pH 7,2 puis les plaquettes recueillies sont diluées à la concentratrion de $2.10^8$ pl./ml.

Les essais sont réalisés sur $4.10^7$ plaquettes en présence de $CaCl_2$ (0,5 mM), de $^{125}I$-fibrinogène (0,1 $\mu M$) et de différentes concentrations de peptide, et la stimulation des plaquettes est provoquée par de l'ADP (5 $\mu M$). Après 15 mn d'incubation et dépôt sur une solution de saccharose à 15% le complexe $^{125}I$-fibrinogène-plaquette est isolé par centrifugation à 12000 t/mn pendant 2 mn.

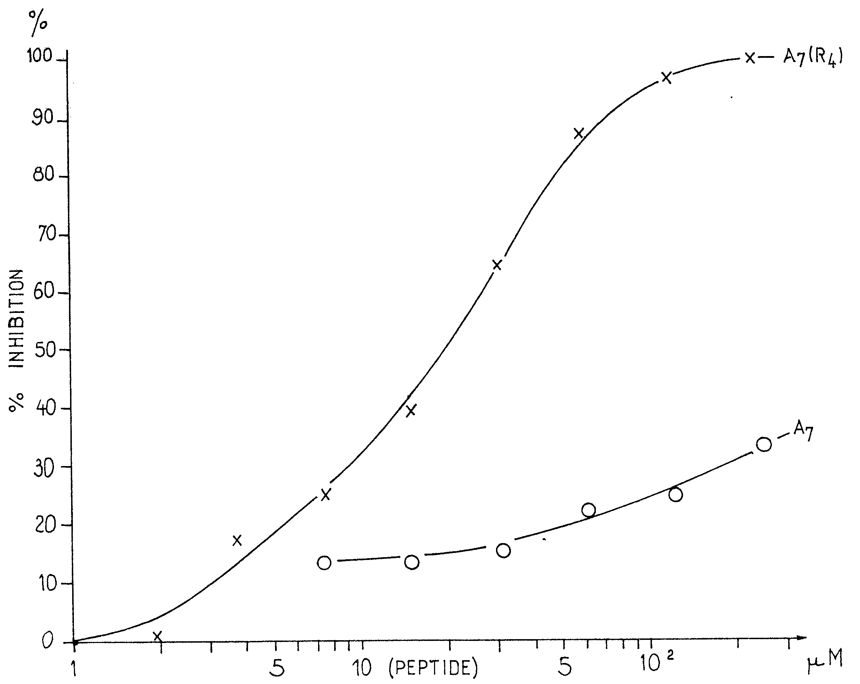

Les résultats pour trois peptides de formule I et les peptides voisins sont donnés sous forme de CI50 dans le tableau II. Ce tableau donne également la séquence des peptides. La Fig. 1 donne les résultats obtenus avec le peptide $A_7$ ($R_4$) de formule I et le peptide de référence $A_7$.

Ces résultats mettent en évidence que les deux peptides de formule I ont une activité inhibitrice sur la liaison plaquette fibrogène bien supérieure à celle du peptide connu $H_{12}$ et à celle du peptide $A_7$ très voisin du peptide $A^7$ ($R_4$).

## 2. Inhibition de l'agrégation plaquettaire

L'effet des peptides synthétiques sur l'agrégation plaquettaire a été étudié sur des plaquettes isolées comme précédemment pour l'étude de la liaison au fibrinogène. La stimulation des plaquettes est également obtenue par l'ADP 5 $\mu M$ et l'essai réalisé en présence de fibrinogène 1,1 $\mu M$ et de $CaCl_2$ 0,5 $\mu M$.

Les résultats sont donnés dans le tableau II et mettent en évidence une activité inhibitrice sur l'agrégation plaquettaire des peptides de formule I nettement supérieure à celle des peptides $H_{12}$ et $A_7$.

## Tableau II

| Peptide | Nomenclature | Inhibition IC50 (µM) | |
|---|---|---|---|
| | | Fixation du Fibrinogène | Agrégation |
| His . His . Leu . Gly . Gly . Ala . Lys . Gla . Ala . Gly . Asp . Val . | H 12 | 150 ± 50 | = 500 |
| Ala ----------------------------- Val | A 7 | - | 500 / 700 |
| Ala ----------- Arg ----------- Val | $A_7(R_4)$ | 20 | 100/200 |
| Lys ------ Arg ---------- Val | $K_6(R_4)$ | 20 | 100/200 |
| Lys ----- Arg -------- Phe | $K_6(R_4F_1)$ | 40 | 50 |

Les dérivés peptidiques de formule I peuvent être utilisés notamment pour le traitement et la prévention des thromboses, en particulier dans les états préthrombotiques pour bloquer l'agrégation plaquettaire.

Les dérivés peptidiques de formule I se sont avérés en outre exercer un effet inhibiteur sur le développement des métastases.

Inhibition du développement des métastases du poumon.

On a utilisé la tumeur $M_4$, une sous-lignée obtenue par sélection d'un sarcome induit par le benzopyrène chez la souris C57BL/6 (Mantovani Int. J. Cancer, 22, 741, 1978).

Les cellules $M_4$ ont été désagrégées avec de la DNAse et de la collagènase pendant 30 minutes à 37°C. On a lavé les cellules deux fois avec du PBS et on les a remis en suspension dans le même tampon à la concentration de $10^6$ cellules/ml. On a injecté lentement dans le veine de la queue de souris C57BL/6 0,2 ml de PBS contenant différentes concentrations de peptide et $10^5$ cellules $M_4$/souris.

Au bout de 15 jours on a tué les souris et excisé les poumons, et on les a fixés avec une solution de Bouin. On a compté visuellement les colonies. Les résultats obtenus avec le peptide KQRGDF sont donnés dans le tableau suivant.

| K Q R G D F mg/souris | Nombre de colonie |
|---|---|
| 3,0 | 0 |
| 1,5 | 0,2 |
| 0,75 | 25,3 ± 9,4 |
| PBS | 117,5 ± 14,4 |

Ces résultats mettent en évidence une protection contre le développement des métastases.

Les dérivés peptidiques de formule I peuvent également exercer un effet inhibiteur sur
- l'adhésion des plaquettes sanguines aux cellules endothéliales des parois vasculaires ou du sous endothélium.
- l'athérogénèse

7

- la réponse inflammatoire

Les compositions thérapeutiques selon l'invention peuvent être administrées à l'homme ou aux animaux par voie orale ou parentérale.

Elles peuvent être sous la forme de préparation solides, semi-solides ou liquides. Comme exemples, on peut citer les comprimés, les gélules, les solutions ou les suspensions injectables.

Dans ces compositions le principe actif est généralement mélangé avec un ou plusieurs excipients pharmaceutiquement acceptables habituels bien connus de l'homme de l'art.

Les compositions thérapeutiques peuvent contenir notamment de 1 à 60% en poids de principe actif.

La quantité de principe actif administrée dépend évidemment du patient qui est traité, de la voie d'administration et de la sévérité de la maladie. Elle est généralement de 1 à 500 mg.

<u>ANNEXE</u>

Abréviations usuelles de la chimie des peptides

BHA : benzylhydrylamine (résine)
Boc : tert-butyloxycarbonyle
Bpoc : 2-(4-biphénylyl)propyl(-2)oxycarbonyle
Bzl : benzyle
Clz : 2-chlorobenzyloxycarbonyle
DCC : dicyclohexylcarbodiimide
DCM : dichlorométhane
DMF : diméthylformamide
EDC : N-ethyl-N'-(3-diméthylaminopropyl) carbodiimide
EEDQ : N-ethyloxycarbonyl-2-ethyloxy-1,2-dihydroquinoline ethyloxycarbonyl-2-ethyloxy-1,2-dihydroquinoline
Fmoc : 9-fluorénylméthyloxycarbonyle
HOBT : 1-hydroxybenzotriazole
MBHA : 4-méthylbenzhydrylamine (résine)
TDM : N,N,N',N', tétraméthyl-4-4' - diaminodiphénylméthane
TFA : acide trifluoroacétique
Tos : tosyle
Xan : xanthyle

## Symboles des acides aminés

| | | |
|---|---|---|
| A | Ala | alanine |
| C | Cys | cystéine |
| D | Asp | acide aspartique |
| E | Glu | acide glutamique |
| F | Phe | phénylalanine |
| G | Gly | glycine |
| H | His | histidine |
| I | Ile | isoleucine |
| K | Lys | lysine |
| L | Leu | leucine |
| M | Met | méthionine |
| N | Asn | asparagine |
| P | Pro | proline |
| Q | Gln | glutamine |
| R | Arg | arginine |
| S | Ser | serine |
| T | Thr | thréonine |
| V | Val | valine |
| W | Trp | tryptophane |
| Y | Tyr | tyrosine |

## Revendications

1. Dérivés peptidiques de formule

X - Lys - Gln - Y - Gly - Asp - Z - W    (I)

dans laquelle X représente l'hydrogène, un résidu d'acide aminé, un groupement de formule DE- où D représente un groupe N-protecteur physiologiquement acceptable, ou l'hydrogène, et où E représente une simple liaison ou un résidu d'acide aminé,

Y représente un résidu L-arginyle, ou D-arginyle,

Z représente un résidu L-valyle, D-valyle, L-phénylalanyle, D-phénylalanyle, L-séryle, ou D-séryle,

W représente un groupe OH, $NH_2$, $O-R^1$ avec $R^1$ représentant un radical alkyle en $C_1$ à $C_4$ $NHR^2$ avec $R^2$ représentant un radical alkyle en $C_1$ à $C_4$, ou un résidu d'acide aminé.

2. Peptide de formule

H - Ala - Lys - Gln - Arg - Gly - Asp - Val - OH

3. Peptide de formule

H - Lys - Gln - Arg - Gly - Asp - Val - OH

4. Peptide de formule

H - Lys - Gln - Arg - Gly - Asp - Phe - OH

5. Composition thérapeutique, caractérisé en ce qu'elle contient à titre de principe actif un dérivé peptidique selon l'une quelconque des revendications 1 à 4.

6. Agent de diagnostic, caractérisé en ce qu'il contient un dérivé peptidique selon l'une quelconque des revendications 1 à 4.

7. Agent de diagnostic, caractérisé en ce qu'il contient un dérivé peptidique tel que défini à la revendication 1.

**Revendications pour l'Etat contractant suivant: GR**

1. Dérivés peptidiques de formule

X - Lys - Gln - Y - Gly - Asp - Z - W     (I)

dans laquelle X représente l'hydrogène, un résidu d'acide aminé, un groupement de formule DE- où D représente un groupe N-protecteur physiologiquement acceptable, ou l'hydrogène, et où E représente une simple liaison ou un résidu d'acide aminé,

Y représente un résidu L-arginyle, ou D-arginyle,

Z représente un résidu L-valyle, D-valyle, L-phénylalanyle, D-phénylalanyle, L-séryle, ou D-séryle,

W représente un groupe OH, $NH_2$, O-$R^1$ avec $R^1$ représentant un radical alkyle en $C_1$ à $C_4$ NH$R^2$ avec $R^2$ représentant un radical alkyle en $C_1$ à $C_4$, ou un résidu d'acide aminé.

2. Peptide de formule

H - Ala - Lys - Gln - Arg - Gly - Asp - Val - OH

3. Peptide de formule

H - Lys - Gln - Arg - Gly - Asp - Val - OH

4. Peptide de formule

H - Lys - Gln - Arg - Gly - Asp - Phe - OH

5. Procédé de préparation d'une composition thérapeutique, caractérisé en ce que l'on met un dérivé peptidique tel que défini à la revendication 1 sous une forme pharmaceutiquement acceptable.

6. Utilisation d'un dérivé peptidique tel que défini à la revendication 1 pour la préparation d'une composition pharmaceutique.

7. Agent de diagnostic, caractérisé en ce qu'il contient un dérivé peptidique tel que défini à la revendication 1.

**Revendications pour l'Etat contractant suivant: ES**

1. Procédé de préparation de dérivés peptidiques de formule

X- Lys - Gln - Y - Gly - Asp - Z - W     (I)

dans laquelle X représente l'hydrogène, un résidu d'acide aminé, un groupement de formule DE- où D représente un groupe N-protecteur physiologiquement acceptable, ou l'hydrogène, et où E représente une simple liaison ou un résidu d'acide aminé,

Y représente un résidu L-arginyle, ou D-arginyle,

Z représente un résidu L-valyle, D-valyle, L-phénylalanyle, D-phénylalanyle, L-séryle, ou D-séryle,

W représente un groupe OH, $NH_2$, O-$R^1$ avec $R^1$ représentant un radical alkyle en $C_1$ à $C_4$, NH$R^2$ avec $R^2$ représentant un radical alkyle en $C_1$ à $C_4$, ou un résidu d'acide aminé, caractérisé en ce que l'on prépare ces dérivés par synthèse peptidique.

2. Procédé selon la revendication 1 caractérisé en ce que l'on prépare un peptide de formule

H - Ala - Lys - Gln - Arg - Gly - Asp - Val - OH

3. Procédé selon la revendication 1 caractérisé en ce que l'on prépare un peptide de formule

H - Lys - Gln - Arg - Gly - Asp - Val - OH

4. Procédé selon la revendication 1 caractérisé en ce que l'on prépare un peptide de formule

H - Lys - Gln - Arg - Gly - Asp - Phe - OH

5. Procédé de préparation d'une composition thérapeutique, caractérisé en ce que l'on met un dérivé peptidique tel que défini à la revendication 1 sous une forme pharmaceutiquement acceptable.

6. Utilisation d'un dérivé peptidique tel que défini à la revendication 1 pour la préparation d'une composition pharmaceutique.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | EP-A-0 220 957 (SCRIPPS CLINIC AND RESEARCH FOUNDATION) <br> * En entier * <br> --- | 1-5 | C 07 K 7/06 <br> A 61 K 37/02 <br> C 12 Q 1/56 <br> G 01 N 33/58 |
| X | PROC. NATL. ACAD. SCI., vol. 83, 1986, pages 5708-5712, US; Z.M. RUGGERI et al.: "Inhibition of platelet function with synthetic peptides designed to be high-affinity antagonists of fibrinogen binding to platelets" <br> * En entier * <br> ----- | 1,5 | |

DOMAINES TECHNIQUES RECHERCHES (Int. Cl.4)

C 07 K 7/00
A 61 K 37/00

Le présent rapport a été établi pour toutes les revendications

| Lieu de la recherche | Date d'achèvement de la recherche | Examinateur |
|---|---|---|
| LA HAYE | 03-10-1988 | RAJIC M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)